# EUROPEAN PATENT SPECIFICATION

(11) **EP 1 915 130 B1**
(45) Date of publication and mention of the grant of the patent: **13.07.2016**
(21) Application number: 06800936.4
(22) Date of filing: 08.08.2006
(51) Int. Cl.: A61K 9/08

(54) **LIQUID FORMULATIONS**
FLÜSSIGE ZUBEREITUNGEN
PREPARATIONS LIQUIDES

(30) Priority: 09.08.2005 US 706820 P
(43) Date of publication of application: 30.04.2008
(73) Proprietor: Novartis AG, 4056 Basel (CH)
(72) Inventor: GEORGOUSIS, Vivian, West Caldwell, NJ 07006 (US); TONG, Wei-Qin, Basking Ridge, NJ 07920 (US)
(74) Representative: Roth, Peter Richard
(86) International application number: PCT/US2006/030836
(87) International publication number: WO 2007/021666

(56) References cited:
- EP-A- 1 424 078
- WO-A-03/097028
- WO-A2-2005/032465
- US-A1- 2004 058 894

## Description

The present invention relates to pharmaceutical compositions comprising a sphingosine-1 phosphate receptor modulator or agonist or a pharmaceutically acceptable salt thereof.

Sphingosine-1 phosphate (hereinafter "S1P") is a natural serum lipid. Presently there are eight known S1P receptors, namely S1P1 to S1P8. S1 P receptor modulators or agonists are typically sphingosine analogues, such as 2-substituted 2-amino- propane-1,3-diol or 2-amino-propanol derivatives, e. g. a compound comprising a group of formula X 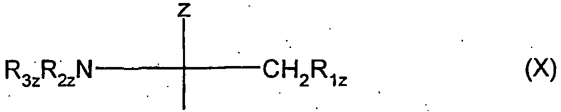 wherein Z is H, C₁₋₆alkyl, C₂₋₆alkenyl, C₂₋₆alkynyl, phenyl, phenyl substituted by OH, C₁₋₆alkyl substituted by 1 to 3 substituents selected from the group consisting of halogen, C₃₋₈cycloalkyl, phenyl and phenyl substituted by OH, or CH₂-R_{4z} wherein R_{4z} is OH, acyloxy or a residue of formula (a) 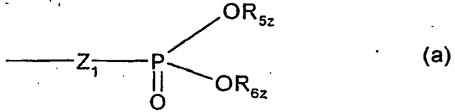
wherein Z₁ is a direct bond or O, preferably O;
each of R_{5z} and R_{6z}, independently, is H, or C₁₋₄alkyl optionally substituted by 1, 2 or 3 halogen atoms;
R_{1z} is OH, acyloxy or a residue of formula (a); and each of R_{2z} and R_{3z} independently, is H, C₁₋₄alkyl or acyl.

Group of formula X is a functional group attached as a terminal group to a moiety which may be hydrophilic or lipophilic and comprise one or more aliphatic, alicyclic, aromatic and/or heterocyclic residues, to the extent that the resulting molecule wherein at least one of Z and R_{1z} is or comprises a residue of formula (a), signals as an agonist at one of more sphingosine-1-phosphate receptor.

S1 P receptor modulators or agonists are compounds which signal as agonists at one or more sphingosine-1 phosphate receptors, e.g. S1P1 to S1P8. Agonist binding to a S1P receptor may e.g. result in dissociation of intracellular heterotrimeric G-proteins into Gα-GTP and Gβγ-GTP, and/or increased phosphorylation of the agonist-occupied receptor and activation of downstream signaling pathways/kinases.

The binding affinity of S1P receptor agonists or modulators to individual human S1P receptors may be determined in following assay:
S1P receptor agonist or modulator activities of compounds are tested on the human S1 P receptors S1P₁, S1P₂, S1P₃, S1P₄ and S1P₅. Functional receptor activation is assessed by quantifying compound induced GTP [γ-³⁵S] binding to membrane protein prepared from transfected CHO or RH7777 cells stably expressing the appropriate human S1P receptor. The assay technology used is SPA (scintillation proximity based assay). Briefly, DMSO dissolved compounds are serially diluted and added to SPA- bead (Amersham-Pharmacia) immobilised S1P receptor expressing membrane protein (10-20µg/well) in the presence of 50 mM Hepes, 100 mM NaCl, 10 mM MgCl₂, 10 µM GDP, 0.1% fat free BSA and 0.2 nM GTP [γ-³⁵S] (1200 Ci/mmol). After incubation in 96 well microtiterplates at RT for 120 min, unbound GTP [γ-³⁵S] is separated by a centrifugation step. Luminescence of SPA beads triggered by membrane bound GTP [γ-³⁵S] is quantified with a TOPcount plate reader (Packard). EC₅₀s are calculated using standard curve fitting software. In this assay, the S1P receptor modulators or agonists preferably have a binding affinity to S1P receptor <50 nM.

Preferred S1P receptor agonists or modulators are e.g. compounds which in addition to their S1P binding properties also have accelerating lymphocyte homing properties, e.g. compounds which elicit a lymphopenia resulting from a re-distribution, preferably reversible, of lymphocytes from circulation to secondary lymphatic tissue, without evoking a generalized immunosuppression. Naïve cells are sequestered; CD4 and CD8 T-cells and B-cells from the blood are stimulated to migrate into lymph nodes (LN) and Peyer's patches (PP).

The lymphocyte homing property may be measured in following Blood Lymphocyte Depletion assay:
A S1P receptor agonist or modulator or the vehicle is administered orally by gavage to rats. Tail blood for hematological monitoring is obtained on day -1 to give the baseline individual values, and at 2, 6, 24, 48 and 72 hours after application. In this assay, the S1P receptor agonist or modulator depletes peripheral blood lymphocytes, e.g. by 50%, when administered at a dose of e.g. < 20 mg/kg.

Examples of appropriate S1P receptor modulators or agonists are, for example:
- Compounds as disclosed in EP627406A1, e.g. a compound of formula I 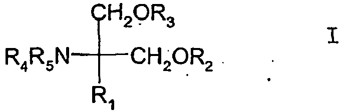 wherein R₁ is a straight- or branched (C₁₂₋₂₂)chain
- which may have in the chain a bond or a hetero atom selected from a double bond, a triple bond, O, S, NR₆, wherein R₆ is H, C₁₋₄alkyl, aryl-C₁₋₄alkyl, acyl or (C₁₋₄alkoxy)carbonyl, and carbonyl, and/or
   - which may have as a substituent C₁₋₄alkoxy, C₂₋₄alkenyloxy, C₂₋₄alkynyloxy, arylC₁₋₄alkyl-oxy, acyl, C₁₋₄alkylamino, C₁₋₄alkylthio, acylamino, (C₁₋₄alkoxy)carbonyl, (C₁₋₄alkoxy)-carbonylamino, acyloxy, (C₁₋₄alkyl)carbamoyl, nitro, halogen, amino, hydroxyimino, hydroxy or carboxy; or
      R₁ is
- a phenylalkyl wherein alkyl is a straight- or branched (C₆₋₂₀)carbon chain; or
- a phenylalkyl wherein alkyl is a straight- or branched (C₁₋₃₀)carbon chain wherein said phenylalkyl is substituted by
- a straight- or branched (C₆₋₂₀)carbon chain optionally substituted by halogen,
- a straight- or branched (C₆₋₂₀)alkoxy chain optionally substitued by halogen,
- a straight- or branched (C₆₋₂₀)alkenyloxy,
- phenyl-C₁₋₁₄alkoxy, halophenyl-C₁₋₄alkoxy, phenyl-C₁₋₁₄alkoxy-C₁₋₁₄alkyl, phenoxy-C₁₋₄alkoxy or phenoxy-C₁₋₄alkyl,
- cycloalkylalkyl substituted by C₆₋₂₀alkyl,
- heteroarylalkyl substituted by C₆₋₂₀alkyl,
- heterocyclic C₆₋₂₀alkyl or
- heterocyclic alkyl substituted by C₂₋₂₀alkyl,
and wherein
the alkyl moiety may have
- in the carbon chain, a bond or a heteroatom selected from a double bond, a triple bond, O, S, sulfinyl, sulfonyl, or NR₆, wherein R₆ is as defined above, and
- as a substituent C₁₋₄alkoxy, C₂₋₄alkenyloxy, C₂₋₄alkynyloxy, arylC₁₋₄alkyloxy, acyl, C₁₋₄alkylamino, C₁₋₄alkylthio, acylamino, (C₁₋₄alkoxy)carbonyl, (C₁₋₄alkoxy)carbonylamino, acyloxy, (C₁₋₄alkyl)carbamoyl, nitro, halogen, amino, hydroxy or carboxy, and
   each of R₂, R₃, R₄ and R₅, independently, is H, C₁₋₄ alkyl or acyl or a pharmaceutically acceptable salt or hydrate thereof;
- Compounds as disclosed in EP 1002792A1, e.g. a compound of formula II 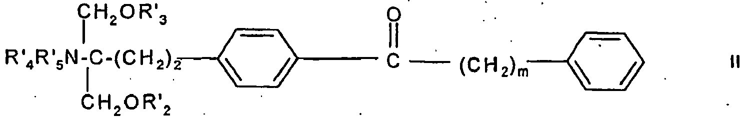 wherein m is 1 to 9 and each of R'₂, R'₃, R'₄ and R'₅, independently, is H, C₁₋₆alkyl or acyl, or a pharmaceutically acceptable salt or hydrate thereof;
- Compounds as disclosed in EP0778263 A1, e.g. a compound of formula III 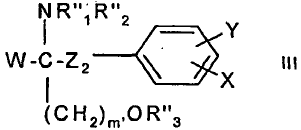 wherein W is H; C₁₋₆alkyl, C₂₋₆alkenyl or C₂₋₆alkynyl; unsubstituted or by OH substituted phenyl; R"₄O(CH₂)ₙ; or C₁₋₆alkyl substituted by 1 to 3 substituents selected from the group consisting of halogen, C₃₋₈cycloalkyl, phenyl and phenyl substituted by OH;
   X is H or unsubstituted or substituted straight chain alkyl having a number p of carbon atoms or unsubstituted or substituted straight chain alkoxy having a number (p-1) of carbon atoms, e.g. substituted by 1 to 3 substitutents selected from the group consisting of C₁₋₆alkyl, OH, C₁₋₆alkoxy, acyloxy, amino, C₁₋₆alkylamino, acylamino, oxo, haloC₁₋₆alkyl, halogen, unsubstituted phenyl and phenyl substituted by 1 to 3 substituents selected from the group consisting of C₁₋₆alkyl, OH, C₁₋₆alkoxy, acyl, acyloxy, amino, C₁₋₆alkylamino, acylamino, haloC₁₋₆alkyl and halogen; Y is H, C₁₋₆alkyl, OH, C₁₋₆alkoxy, acyl, acyloxy, amino, C₁₋₆alkylamino, acylamino, haloC₁₋₆alkyl or halogen, Z₂ is a single bond or a straight chain alkylene having a number or carbon atoms of q,
   each of p and q, independently, is an integer of 1 to 20, with the proviso of 6≤p+q≤23, m' is 1, 2 or 3, n is 2 or 3,
   each of R"₁, R"₂, R"₃ and R"₄, independently, is H, C₁₋₄alkyl or acyl,
   or a pharmaceutically acceptable salt or hydrate thereof,
- Compounds as disclosed in WO02/18395, e.g. a compound of formula IVa or IVb 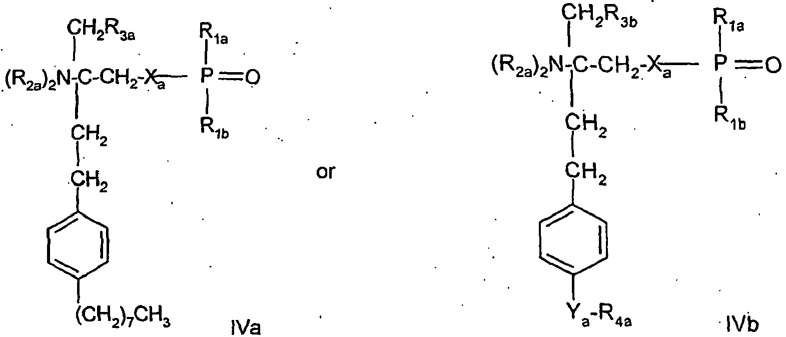 wherein Xₐ is O, S, NR₁ₛ or a group -(CH₂)ₙₐ-, which group is unsubstituted or substituted by 1 to 4 halogen; nₐ is 1 or 2, R₁ₛ is H or (C₁₋₄)alkyl, which alkyl is unsubstituted or substituted by halogen; R₁ₐ is H, OH, (C₁₋₄)alkyl or O(C₁₋₄)alkyl wherein alkyl is unsubstituted or substituted by 1 to 3 halogen; R_{1b} is H, OH or (C₁₋₄)alkyl, wherein alkyl is unsubstituted or substituted by halogen; each R₂ₐ is independently selected from H or (C₁₋₄)alkyl, which alkyl is unsubstituted or substitued by halogen; R₃ₐ is H, OH, halogen or O(C₁₋₄)alkyl wherein alkyl is unsubstituted or substituted by halogen; and R_{3b} is H, OH, halogen, (C₁₋₄)alkyl wherein alkyl is unsubstituted or substituted by hydroxy, or O(C₁₋₄)alkyl wherein alkyl is unsubstituted or substituted by halogen; Yₐ is -CH₂-, -C(O)-, -CH(OH)-, -C(=NOH)-, O or S, and R₄ₐ is (C₄₋₁₄)alkyl or (C₄₋₁₄)alkenyl;
   or a pharmaceutically acceptable salt or hydrate thereof;
- Compounds as disclosed in WO02/06268A1, e.g. a compound of formula V 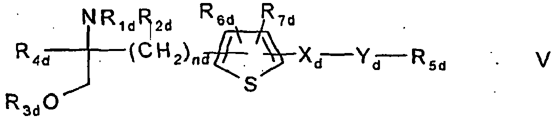 wherein each of R_{1d} and R_{2d}, independently, is H or an amino-protecting group; R_{3d} is hydrogen, a hydroxy-protecting group or a residue of formula 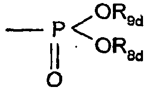
   R_{4d} is C₁₋₄alkyl;
   n_{d} is an integer of 1 to 6;
   X_{d} is ethylene, vinylene, ethynylene, a group having a formula - D-CH₂- (wherein D is carbonyl, - CH(OH)-, O, S or N), aryl or aryl substituted by up to three substitutents selected from group a as defined hereinafter;
   Y_{d} is single bond, C₁₋₁₀alkylene, C₁₋₁₀alkylene which is substituted by up to three substitutents selected from groups a and b, C₁₋₁₀alkylene having O or S in the middle or end of the carbon chain, or C₁₋₁₀alkylene having O or S in the middle or end of the carbon chain which is substituted by up to three substituents selected from groups a and b;
   R_{5d} is hydrogen, C₃₋₆cycloalkyl, aryl, heterocyclic group, C₃₋₆cycloalkyl substituted by up to three substituents selected from groups a and b, aryl substituted by up to three substituents selected from groups a and b, or heterocyclic group substituted by up to three substituents selected from groups a and b;
   each of R_{6d} and R_{7d}, independently, is H or a substituent selected from group a;
   each of R_{8d} and R_{9d}, independently, is H or C₁₋₄alkyl optionally substituted by halogen;
   <group a > is halogen, lower alkyl, halogeno lower alkyl, lower alkoxy, lower alkylthio, carboxyl, lower alkoxycarbonyl, hydroxy, lower aliphatic acyl, amino, mono-lower alkylamino, di-C₁₋₄alkylamino, acylamino, cyano or nitro; and
   <group b > is C₃₋₆cycloalkyl, aryl or heterocyclic group, each being optionally substituted by up to three substituents selected from group a;
      with the proviso that when R_{5d} is hydrogen, Y_{d} is a either a single bond or linear C₁₋₁₀ alkylene, or a pharmacologically acceptable salt, ester or hydrate thereof;
- Compounds as disclosed in JP-14316985 (JP2002316985), e.g. a compound of formula VI 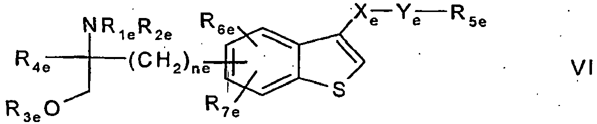 wherein R₁ₑ,R₂ₑ,R₃ₑ,R₄ₑ,R₅ₑ,R₆ₑ,R₇ₑ,nₑ, Xₑ and Yₑ are as disclosed in JP-14316985;
   or a pharmacologically acceptable salt, ester or hydrate thereof;
- Compounds as disclosed in WO 03/29184 and WO 03/29205, e.g. compounds of formula IX 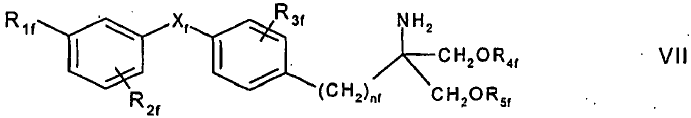
   wherein X_{f} is O, S, SO or SO₂ R_{1f} is halogen, trihalomethyl, OH, C₁₋₇alkyl, C₁₋₄alkoxy, trifluoromethoxy, phenoxy, cyclohexylmethyloxy, pyridylmethoxy, cinnamyloxy, naphthylmethoxy, phenoxymethyl, CH₂-OH, CH₂-CH₂-OH, C₁₋₄alkylthio, C₁₋₄alkylsulfinyl, C₁₋₄alkylsulfonyl, benzylthio, acetyl, nitro or cyano, or phenyl, phenylC₁₋₄alkyl or phenyl-C₁₋₄alkoxy each phenyl group thereof being optionally substituted by halogen, CF₃, C₁₋₄alkyl or C₁₋₄alkoxy;
   R_{2f} is H, halogen, trihalomethyl, C₁₋₄alkoxy, C₁₋₇alkyl, phenethyl or benzyloxy;
   R_{3f} H, halogen, CF₃, OH, C₁₋₇alkyl, C₁₋₄alkoxy, benzyloxy or C₁₋₄alkoxymethyl;
   each of R_{4f} and R_{5f}, independently is H or a residue of formula 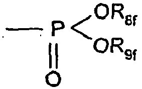
   wherein each of R_{8f} and R_{9f}, independently, is H or C₁₋₄alkyl optionally substituted by halogen;and
   n*_{f}* is an integer from 1 to 4;
   or a pharmacological salt, solvate or hydrate thereof;
- Compounds as disclosed in WO03/062252A1, e.g. a compound of formula VIII 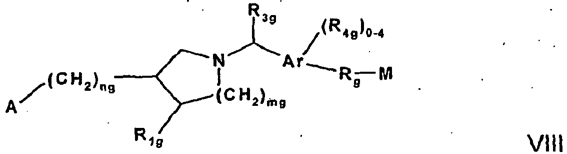 wherein
   Ar is phenyl or naphthyl; each of m_{g} and n_{g} independently is 0 or 1; A is selected from COOH, PO₃H₂, PO₂H, SO₃H, PO(C₁₋₃alkyl)OH and 1*H*-tetrazol-5-yl; each of R_{1g} and R_{2g} independently is H, halogen, OH, COOH or C₁₋₄alkyl optionally substituted by halogen; R_{3g} is H or C₁₋₄alkyl optionally substituted by halogen or OH; each R_{4g} independently is halogen, or optionally halogen substituted C₁₋₄alkyl or C₁₋₃alkoxy; and each of R_{g} and M has one of the significances as indicated for B and C, respectively, in WO03/062252A1;
   or a pharmacologically acceptable salt, solvate or hydrate thereof;
- Compounds as disclosed in WO 03/062248A2, e.g. a compound of formula IX 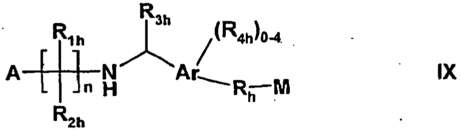
   wherein Ar is phenyl or naphthyl; n is 2,3 or 4; A is COOH, 1 H-tetrazol-5-yl, PO₃H₂, PO₂H₂, - SO₃H or PO(R₅ₕ)OH wherein R₅ₕ is selected from C₁₋₄alkyl, hydroxyC₁₋₄alkyl, phenyl, -CO-C₁₋₃alkoxy and -CH(OH)-phenyl wherein said phenyl or phenyl moiety is optionally substituted; each of R₁ₕ and R₂ₕ independently is H, halogen, OH, COOH, or optionally halogeno substituted C₁₋₆alkyl or phenyl; R₃ₕ is H or C₁₋₄alkyl optionally substituted by halogen and/ OH; each R₄ₕ independently is halogeno, OH, COOH, C₁₋₄alkyl, S(O)_{0,1 or2}C₁₋₃alkyl, C₁₋₃alkoxy, C₃₋₆cycloalkoxy, aryl or aralkoxy, wherein the alkyl portions may optionally be substituted by 1-3 halogens; and each of Rₕ and M has one of the significances as indicated for B and C, respectively, in WO03/062248A2
   or a pharmacologically acceptable salt, solvate or hydrate thereof.
- Compounds as disclosed in WO 04/026817A, e.g. compounds of formula X 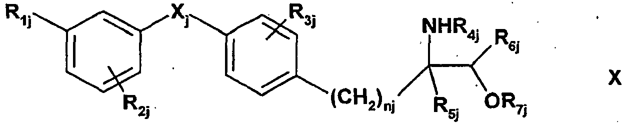 wherein
   R₁ⱼ is halogen, trihalomethyl, C₁₋₄alkyl, C₁₋₄alkoxy, C₁₋₄alkylthio, C₁₋₄alkylsulifinyl, C₁₋₄alkylsulfonyl, aralkyl, optionally substituted phenoxy or aralkyloxy, R₂ⱼ is H, halogen, trihalo-methyl, C₁₋₄alkyl, C₁₋₄alkoxy, aralkyl or aralkyloxy, R₃ⱼ is H, halogen, CF₃, C₁₋₄alkyl, C₁₋₄alkoxy, C₁₋₄alkylthio or benzyloxy, R₄ⱼ is H, C₁₋₄alkyl, phenyl, optionally substituted benzyl or benzoyl, or lower aliphatic C ₁₋₅acyl, R₅ⱼ is H, monohalomethyl, C₁₋₄alkyl, C₁₋₄alkoxymethyl, C₁₋₄alkylthiomethyl, hydroxyethyl, hydroxypropyl, phenyl, aralkyl, C₂₋₄alkenyl or -alkynyl, each of R₆ⱼ and R₇ⱼ, independently, is H or C₁₋₄alkyl, Xⱼ is O, S, SO or SO₂ and nⱼ is an integer of 1 to 4, or a pharmacologically acceptable salt, solvate or hydrate thereof.
- Compounds as disclosed in WO 04/103306A, WO 05/000833, WO 05/103309 or WO 05/113330, e.g. compounds of formula XIa or XIb 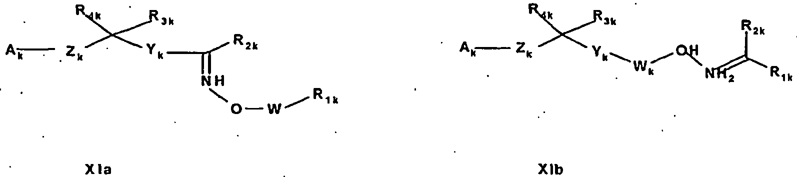 wherein
   Aₖ is COOR₅ₖ, OPO(OR₅ₖ)₂, PO(OR₅ₖ)₂, SO₂OR₅ₖ, POR₅ₖOR₅ₖ or 1*H*-tetrazol-5-yl, R₅ₖ being H or C₁₋₆alkyl;
   Wₖ is a bond, C₁₋₃alkylene or C₂₋₃alkenylene;
   Yₖ is C₆₋₁₀aryl or C₃₋₉heteroaryl, optionally substituted by 1 to 3 radicals selected from halogene, OH, NO₂, C₁₋₆alkyl, C₁₋₆alkoxy; halo-substituted C₁₋₆alkyl and halo-substituted C₁₋₆alkoxy;
   Zₖ is a heterocyclic group as indicated in WO 04/103306A, e.g. azetidine;
   R₁ₖ is C₆₋₁₀aryl or C₃₋₉heteroaryl, optionally substituted by C₁₋₆alkyl, C₆₋₁₀aryl, C₆₋₁₀arylC₁₋₄alkyl, C₃₋₉heteroaryl, C₃₋₉heteroarylC₁₋₄alkyl, C₃₋₈cycloalkyl, C₃₋₈cycloalkylC₁₋₄alkyl, C₃₋₈heterocycloalkyl or C₃₋₈heterocycloalkylC₁₋₄alkyl; wherein any aryl, heteroaryl, cycloalkyl or heterocycloalkyl of R₁ₖ may be substituted by 1 to 5 groups selected from halogen, C₁₋₆alkyl, C₁₋₆alkoxy and halo substituted-C₁₋₆alkyl or -C₁₋₆alkoxy;
   R₂ₖ is H, C₁₋₆alkyl, halo substituted C₁₋₆alkyl, C₂₋₆alkenyl or C₂₋₆alkynyl: and
   each of R₃ₖ or R₄ₖ, independently, is H, halogen, OH, C₁₋₆alkyl, C₁₋₆alkoxy or halo substituted C₁₋₆alkyl or C₁₋₆alkoxy;
   and the N-oxide derivatives thereof or prodrugs thereof,
   or a pharmacologically acceptable salt, solvate or hydrate thereof.

The compounds of formulae I to XIb may exist in free or salt form. Examples of pharmaceutically acceptable salts of the compounds of the formulae I to VI include salts with inorganic acids, such as hydrochloride, hydrobromide and sulfate, salts with organic acids, such as acetate, fumarate, maleate, benzoate, citrate, malate, methanesulfonate and benzenesulfonate salts, or, when appropriate, salts with metals such as sodium, potassium, calcium and aluminium, salts with amines, such as triethylamine and salts with dibasic amino acids, such as lysine. Examples of pharmaceutically acceptable salts of the compounds of the formulae VII and X include salts with inorganic acids, such as hydrochloride and hydrobromide, salts with organic acids, such as acetate, trifluoroacetate, citrate, tartrate, methanesulfonate and benzenesulfonate salts. The compounds and salts of the combination of the present invention encompass hydrate and solvate forms.

Acyl as indicated above may be a residue R_{y}-CO- wherein R_{y} is C₁₋₆alkyl, C₃₋₆cycloalkyl, phenyl or phenyl-C₁₋₄alkyl. Unless otherwise stated, alkyl, alkoxy, alkenyl or alkynyl may be straight or branched.

Aryl may be phenyl or naphthyl, preferably phenyl.

When in the compounds of formula I the carbon chain as R₁ is substituted, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy.

Preferred compounds of formula I are those wherein R₁ is C₁₃₋₂₀alkyl, optionally substituted by nitro, halogen, amino, hydroxy or carboxy, and, more preferably those wherein R₁ is phenylalkyl substituted by C₆₋₁₄-alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

In the above formula of V "heterocyclic group" represents a 5- to 7 membered heterocyclic group having 1 to 3 heteroatoms selected from S, O and N. Examples of such heterocyclic groups include the heteroaryl groups indicated above, and heterocyclic compounds corresponding to partially or completely hydrogenated heteroaryl groups, e.g. furyl, thienyl, pyrrolyl, azepinyl, pyrazolyl, imidazolyl, oxazolyl, isoxazolyl, thiazolyl, isothiazolyl, 1,2,3-oxadiazolyl, triazolyl, tetrazolyl, thiadiazolyl, pyranyl, pyridyl, pyridazinyl, pyrimidinyl, pyrazinyl, tetrahydropyranyl, morpholinyl, thiomorpholinyl, pyrrolidinyl, pyrrolyl, imidazolidinyl, pyrazolidinyl, piperidinyl, piperazinyl, oxazolidinyl, isoxazolidinyl, thiazolidinyl or pyrazolidinyl. Preferred heterocyclic groups are 5-or 6-membered heteroaryl groups and the most preferred heteocyclic group is a morpholinyl, thiomorpholinyl or piperidinyl group.

A preferred compound of formula I is 2-amino-2-tetradecyl-1,3-propanediol. A particularly preferred S1P receptor agonist of formula I is FTY720, i.e. 2-amino-2-[2-(4-octylphenyl) ethyl]propane-1,3-diol in free form or in a pharmaceutically acceptable salt form (referred to hereinafter as Compound A), e.g. the hydrochloride, as shown:

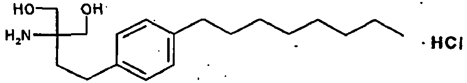

A preferred compound of formula II is the one wherein each of R'₂ to R'₅ is H and m is 4, i.e. 2-amino-2-{2-[4-(1-oxo-5-phenylpentyl)phenyl]ethyl}propane-1,3-diol, in free form or in pharmaceutically acceptable salt form (referred to hereinafter as Compound B), e.g the hydrochloride.

A preferred compound of formula III is the one wherein W is CH₃, each of R"₁ to R"₃ is H, Z₂ is ethylene, X is heptyloxy and Y is H, i.e. 2-amino-4-(4-heptyloxyphenyl)-2-methyl-butanol, in free form or in pharmaceutically acceptable salt form (referred to hereinafter as Compound C), e.g. the hydrochloride. The R-enantiomer is particularly preferred.

A preferred compound of formula IVa is the FTY720-phosphate (R₂ₐ is H, R₃ₐ is OH, Xₐ is O, R₁ₐ and R_{1b} are OH). A preferred compound of formula IVb is the Compound C-phosphate (R₂ₐ is H, R_{3b} is OH, Xₐ is O, R₁ₐ and R_{1b} are OH, Yₐ is O and R₄ₐ is heptyl). A preferred compound of formula V is Compound B-phosphate.

A preferred compound of formula VI is (2R)-2-amino-4-[3-(4-cyclohexyloxybutyl)-benzo[b]thien-6-yl]-2-methylbutan-1-ol.

A preferred compound of formula VII is e.g. 2-amino-2-[4-(3-benzyloxyphenoxy)-2-chlorophenyl]ethyl-1,3-propane-diol, 2-amino-2-[4-(benzyloxyphenylthio)-2-chlorophenyl]ethyl-1,3-propane-diol, 2-amino-2-[4-(3-benzyloxyphenoxy)-2-chlorophenyl]propyl-1,3-propane-diol or 2-amino-2-[4-(benzyloxyphenylthio)-2-chlorophenyl]propyl-1,3-propane-diol.

A preferred compound of formula X is e.g. 2-amino-4-[4-(3-benzyloxyphenylthio)-2-chlorophenyl]-2-methylbutane-1-ol or 2-amino-4-[4-(3-benzyloxyphenylthio)-2-chlorophenyl]-2-ethylbutane-1-ol.

A preferred compound of formula XIa is e.g. 1-{4-[1-(4-cyclohexyl-3-trifluoromethyl-benzyloxyimino)-ethyl]-2-ethyl-benzyl}-azetidine-3-carboxylic acid, or a prodrug thereof.

The various known S1P receptor agonists show structural similarities, which result in related problems in providing a suitable formulation for oral administration.

There is a need for a S1P receptor modulator or agonist containing oral formulation which can easily be swallowed, e.g. by children, patients in a difficult condition to swallow due to their diseases or older patients. A liquid dosage is especially preferred for such patients because of the ease with which it may be swallowed and thus patients may be more inclined to comply with their medication instruction. Additionally, a liquid formulation provides flexibility in mg/kg dosing.

However, compositions in form of aqueous solutions comprising a S1P receptor modulator or agonist or a pharmaceutically acceptable salt thereof have been observed to lead to crystalline deposits of the drug either shortly after preparation or upon storage.

Furthermore, when formulating a drug for oral administration to pediatric patients, one is limited to a smaller number of suitable excipients, e.g. such a composition should preferably be ethanol-free.

It has now been found that compositions in form of a concentrate for dilution comprising propylene glycol and optionally glycerin are physically stable for extended periods of time, e.g. more than six months at ambient temperature.

Accordingly, the present application provides a concentrate for dilution comprising a S1P receptor modulator or agonist or a pharmaceutically acceptable salt thereof, propylene glycol and optionally glycerin.

The concentrate for dilution of the invention preferably contains about 5 to 20% by weight of S1P receptor agonists, more preferably about 7 to 15%, e.g. about 10% by weight, based on the total weight of the composition.

The amount of glycerin in the concentrate for dilution of the invention typically ranges from 0 to 35%, e.g.1 to 35%, e.g. about 5 to 25% by weight, based on the total weight of the composition.

The amount of propylene glycol in the concentrate for dilution of the invention ranges from 65 to 99%, e.g. 75 to 95% by weight, based on the total weight of the composition.

The ratio of glycerin (when present) to propylene glycol in the concentrate for dilution of the invention typically is between about 5:95 to about 25:75, e.g. about 5:95, 10:90, 15:85, 20:80, 25:75, preferably about 25:75.

Preferably, the concentrate for dilution of the invention shows a flow behavior to allow dosing with a syringe:

The concentrate for dilution of the invention may comprise one or more further excipients e.g. yet another solvent, a flavor and/or a preservative.

Preferably, the concentrates of the present invention are ethanol-free.

Suitable flavor include citrus flavors including cherry, strawberry, grape, punch, tutti-frutti, e.g. as available from Firmenich Inc.. The amount of flavor in the concentrate for dilution of the invention ranges from 0 to 0.5% by weight, based on the total weight of the composition.

Suitable preservative include a hydroxybenzoic acid derivative, e.g. methyl-, propyl- or butyl-paraben. The amount of preservative in the concentrate for dilution of the invention ranges from 0.05 to 0.13% by weight, based on the total weight of the composition.

The concentrate for dilution of the invention may be produced by standard processes; for instance by conventional mixing. Procedures which may be used are known in the art, e.g. those described in L. Lachman et al. The Theory and Practice of Industrial Pharmacy, 3rd Ed, 1986, H. Sucker et al, Pharmazeutische Technologie, Thieme, 1991, Hagers Handbuch der pharmazeutischen Praxis, 4th Ed. (Springer Verlag, 1971) and Remington's Pharmaceutical Sciences, 13th Ed., (Mack Publ., Co., 1970) or later editions.

In one aspect, the present invention relates to a process for producing a concentrate of the invention comprising dissolution of a S1P receptor modulator or agonist or a pharmaceutically acceptable salt thereof and, optionally, another solvent, a flavor and/or a preservative, in a propylene glycol and addition of glycerin.

Prior to administration, the required amount of concentrate of the invention is dosed e.g. with a syringe, and diluted with a vehicle.

Suitable dilution vehicles include water, sparkling water, fruit juices e.g. orange or apple juice, soda such as colas, limeade, and lemonade.

The ratio of concentrate to dilution vehicle may be from 1:1 to more than 1:10; preferably it is more than 1:10.

In another aspect, the present invention also provides a pharmaceutical kit comprising the concentrate and the dilution vehicle.

The pharmaceutical solution so formed may be preferably used immediately or within a short time of being formed, e.g. within four hours.

The concentrates of the present invention or the pharmaceutical solutions resulting from the dilution are useful, either alone or in combination with other active agents, for the treatment and prevention of conditions e.g. as disclosed in US 5,604,229, WO 97/24112, WO 01/01978, US 6,004,565, US 6,274,629 and JP-14316985 for the compounds of formula I, e.g. in WO 03/29184 and WO 03/29205 for the compounds of formula VII, in WO 04/026817A for the compounds of formula X, or in WO 04/103306A, WO 05/000833, WO 05/103309 or WO 05/113330 for the compounds of formulae XIa and XIb, the contents of which are incorporated herein by reference.

In particular, a concentrate of the invention or the pharmaceutical solution resulting from the dilution is useful for:
a) treatment and prevention of organ or tissue transplant rejection, for example for the treatment of the recipients of heart, lung, combined heart-lung, liver, kidney, pancreatic, skin or corneal transplants, and the prevention of graft-versus-host disease, such as sometimes occurs following bone marrow transplantation; particularly in the treatment of acute or chronic allo- and xenograft rejection or in the transplantation of insulin producing cells, e.g. pancreatic islet cells;
b) treatment and prevention of autoimmune disease or of inflammatory conditions, e.g. multiple sclerosis, arthritis (for example rheumatoid arthritis), inflammatory bowel diseases, hepatitis, etc.;
c) treatment and prevention of viral myocarditis and viral diseases caused by viral myocarditis, including hepatitis and AIDS.

The concentrate for dilution or the pharmaceutical solution made therefrom, may be administered to a patient in need of immunosuppression, in an amount which is therapeutically effective, e.g. against a disease or condition which can be treated by administration of the S1P receptor modulator or agonist. The exact amount of S1P receptor modulator or agonist or pharmaceutically acceptable salt thereof to administer can vary widely. The dose may depend on the particular compound, the rate of administration, the strength of the particular concentrate or pharmaceutical solution employed, the nature of the disease or condition being treated, and the sex, age and body weight of the patient. The dose may also depend on the existence, nature and extent of any adverse side-effects that may accompany the administration of the concentrate or pharmaceutical formulation. Typically, a dose of 0.5 to 5 mg of S1P receptor modulator or agonist, e.g. Compound A, may be administered to a child or an adult patient having difficulties to swallow.

The concentrate for dilution or the respective pharmaceutical solution may be used in combination with other immunosuppressant(s), steroid(s) such as prednisolone, methylprednisolone, dexamethasone, hydrocortisone and the like, or nonsteroidal antiinflammatory agent. The administration of a combination of active agents may be simultaneous or consecutive, with either one of the active agents being administered first. The dosage of the active agents of a combination treatment may depend on effectiveness and site of action of each active agent, as well as synergistic effects between the agents used for combination therapy.

A preferred concentrate or pharmaceutical solution for oral administration, is the one comprising Compound A hydrochloride, as S1P receptor modulator, e.g. for use in the treatment of multiple sclerosis.

The invention will now be described with reference to the following specific embodiments, without any limitation.

**Examples 1 to 3**

| | Ex. 1 | Ex. 2 | Ex. 3 |
|---|---|---|---|
| Compound A hydrochloride | 11.12 mg | 11.12 mg | 5.56 mg |
| Glycerin | 250 mg | 10 mg | 350 mg |
| Methyl paraben | ----- | 0.5 mg | ----- |
| Tutti Frutti Flavor | 2.5 mg | 5 mg | 2.5 mg |
| Propylene glycol | qsad 1 ml | qsad 1 ml | qsad 1 ml |

Compound A, the flavor and the preservative, if present, are dissolved in propylene glycol in amounts given in the table. Then glycerin (in an amount as given in the table) is added to said solution.

The composition is stable for at least six months at ambient temperature.

Prior to administration, about 0.05 ml to 1 ml of the concentrate are diluted with about 10 ml or more of water, fruit juice or soda.

By following the procedure as described above, the following compositions may be prepared:

**Examples 4 to 6**

| | Ex. 4 | Ex. 5 | Ex. 6 |
|---|---|---|---|
| Compound A hydrochloride | 2.78 mg | 2.78 mg | 1.39 mg |
| Glycerin | 250 mg | 10 mg | 350 mg |
| Methyl paraben | ----- | 0.5 mg | ----- |
| Tutti Frutti Flavor | 2.5 mg | 5 mg | 2.5 mg |
| Propylene glycol | qsad 1 ml | qsad 1 ml | qsad 1 ml |

The same formulations as disclosed above may be prepared without flavor or with another flavor.

The same formulations as disclosed above may be prepared without glycerin.

## Claims

1. A concentrate for dilution comprising a S1P receptor modulator or agonist or a pharmaceutically acceptable salt thereof, and 65 to 99% by weight of propylene glycol, based on the total weight of the composition.

2. A concentrate according to claim 1 wherein the S1P receptor modulator or agonist is selected from:
- a compound of formula I 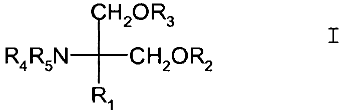 wherein R₁ is
- a phenylalkyl wherein alkyl is a straight- or branched (C₆₋₂₀)carbon chain; or
- a phenylalkyl wherein alkyl is a straight- or branched (C₁₋₃₀)carbon chain wherein said phenylalkyl is substituted by
- a straight- or branched (C₆₋₂₀)carbon chain optionally substituted by halogen,
- a straight- or branched (C₆₋₂₀)alkoxy chain optionally substitued by halogen,
- a straight- or branched (C₆₋₂₀)alkenyloxy,
- phenyl-C₁₋₁₄alkoxy, halophenyl-C₁₋₄alkoxy, phenyl-C₁₋₁₄alkoxy-C₁₋₁₄alkyl, phenoxy-C₁₋₄alkoxy or phenoxy-C₁₋₄alkyl,
- cycloalkylalkyl substituted by C₆₋₂₀alkyl,
- heteroarylalkyl substituted by C₆₋₂₀alkyl,
- heterocyclic C₆₋₂₀alkyl or
- heterocyclic alkyl substituted by C₂₋₂₀alkyl,
and wherein
the alkyl moiety may have
- in the carbon chain, a bond or a heteroatom selected from a double bond, a triple bond, O, S, sulfinyl, sulfonyl, or NR₆, wherein R₆ is as defined above, and
- as a substituent C₁₋₄alkoxy, C₂₋₄alkenyloxy, C₂₋₄alkynyloxy, arylC₁₋₄alkyloxy, acyl, C₁₋₄alkylamino, C₁₋₄alkylthio, acylamino, (C₁₋₄alkoxy)carbonyl, (C₁₋₄alkoxy)carbonylamino, acyloxy, (C₁₋₄alkyl)carbamoyl, nitro, halogen, amino, hydroxy or carboxy, and
each of R₂, R₃, R₄ and R₅, independently, is H, C₁₋₄ alkyl or acyl
or a pharmaceutically acceptable salt or hydrate thereof;
- a compound of formula II 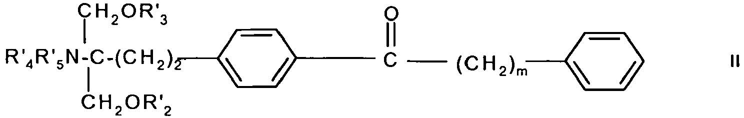 wherein m is 1 to 9 and each of R'₂, R'₃, R'₄ and R'₅, independently, is H, C₁₋₆alkyl or acyl, or a pharmaceutically acceptable salt or hydrate thereof;
- a compound of formula III 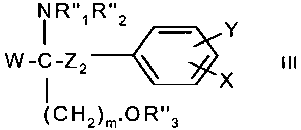
wherein W is H; C₁₋₆alkyl, C₂₋₆alkenyl or C₂₋₆alkynyl; unsubstituted or by OH substituted phenyl; R"₄O(CH₂)ₙ; or C₁₋₆alkyl substituted by 1 to 3 substituents selected from the group consisting of halogen, C₃₋₈cycloalkyl, phenyl and phenyl substituted by OH;
X is H or unsubstituted or substituted straight chain alkyl having a number p of carbon atoms or unsubstituted or substituted by 1 to 3 substitutents selected from the group consisting of C₁₋₆alkyl, OH, C₁₋₆alkoxy, acyloxy, amino, C₁₋₆alkylamino, acylamino, oxo, haloC₁₋₆alkyl, halogen, unsubstituted phenyl and phenyl substituted by 1 to 3 substituents selected from the group consisting of C₁₋₆alkyl, OH, C₁₋₆alkoxy, acyl, acyloxy, amino, C₁₋₆alkylamino, acylamino, haloC₁₋₆alkyl and halogen;
Y is H, C₁₋₆alkyl, OH, C₁₋₆alkoxy, acyl, acyloxy, amino, C₁₋₆alkylamino, acylamino, haloC₁₋₆alkyl or halogen,
Z₂ is a single bond or a straight chain alkylene having a number or carbon atoms of q,
each of p and q, independently, is an integer of 1 to 20, with the proviso of 6≤p+q≤23, m' is 1, 2 or 3, n is 2 or 3,
each of R"₁, R"₂, R"₃ and R"₄, independently, is H, C₁₋₄alkyl or acyl,
or a pharmaceutically acceptable salt or hydrate thereof,
- a compound of formula IVa or IVb 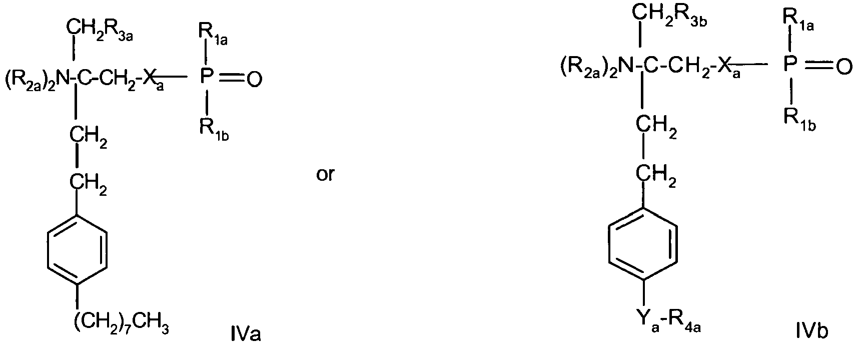
wherein Xₐ is O, S, NR₁ₛ or a group -(CH₂)ₙₐ-, which group is unsubstituted or substituted by 1 to 4 halogen;
nₐ is 1 or 2, R₁ₛ is H or (C₁₋₄)alkyl, which alkyl is unsubstituted or substituted by halogen;
R₁ₐ is H, OH, (C₁₋₄)alkyl or O(C₁₋₄)alkyl wherein alkyl is unsubstituted or substituted by 1 to 3 halogen;
R_{1b} is H, OH or (C₁₋₄)alkyl, wherein alkyl is unsubstituted or substituted by halogen;
each R₂ₐ is independently selected from H or (C₁₋₄)alkyl, which alkyl is unsubstituted or substitued by halogen;
R₃ₐ is H, OH, halogen or O(C₁₋₄)alkyl wherein alkyl is unsubstituted or substituted by halogen; and R_{3b} is H, OH, halogen, (C₁₋₄)alkyl wherein alkyl is unsubstituted or substituted by hydroxy, or O(C₁₋₄)alkyl wherein alkyl is unsubstituted or substituted by halogen;
Yₐ is -CH₂-, -C(O)-, -CH(OH)-, -C(=NOH)-, O or S,
and R₄ₐ is (C₄₋₁₄)alkyl or (C₄₋₁₄)alkenyl;
or a pharmaceutically acceptable salt or hydrate thereof;
- a compound of formula VII 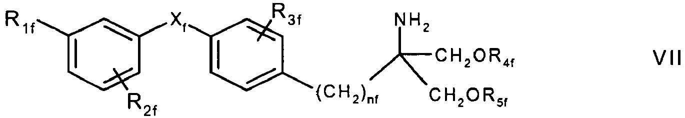
wherein X_{f} is O, S, SO or SO₂
R_{1f} is halogen, trihalomethyl, OH, C₁₋₇alkyl, C₁₋₄alkoxy, trifluoromethoxy, phenoxy, cyclohexylmethyloxy, pyridylmethoxy, cinnamyloxy, naphthylmethoxy, phenoxymethyl, CH₂-OH, CH₂-CH₂-OH, C₁₋₄alkylthio, C₁₋₄alkylsulfinyl, C₁₋₄alkylsulfonyl, benzylthio, acetyl, nitro or cyano, or phenyl, phenylC₁₋₄alkyl or phenyl-C₁₋₄alkoxy each phenyl group thereof being unsubstituted or substituted by halogen, CF₃, C₁₋₄alkyl or C₁₋₄alkoxy;
R_{2f} is H, halogen, trihalomethyl, C₁₋₄alkoxy, C₁₋₇alkyl, phenethyl or benzyloxy;
R_{3f} H, halogen, CF₃, OH, C₁₋₇alkyl, C₁₋₄alkoxy, benzyloxy or C₁₋₄alkoxymethyl;
each of R_{4f} and R_{5f}, independently is H or a residue of formula 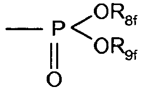
wherein each of R_{8f} and R_{9f}, independently, is H or C₁₋₄alkyl unsubstituted or substituted by halogen;
and n_{f} is an integer from 1 to 4;
or a pharmacological salt, solvate or hydrate thereof;
- a compound of formula X 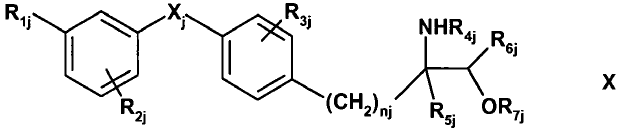 wherein
R₁ⱼ is halogen, trihalomethyl, C₁₋₄alkyl, C₁₋₄alkoxy, C₁₋₄alkylthio, C₁₋₄alkylsulifinyl, C₁₋₄alkylsulfonyl, aralkyl, unsubstituted or substituted by phenoxy or aralkyloxy,
R₂ⱼ is H, halogen, trihalo-methyl, C₁₋₄alkyl, C₁₋₄alkoxy, aralkyl or aralkyloxy,
R₃ⱼ is H, halogen, CF₃, C₁₋₄alkyl, C₁₋₄alkoxy, C₁₋₄alkylthio or benzyloxy,
R₄ⱼ is H, C₁₋₄alkyl, phenyl, unsubstituted or substituted by benzyl or benzoyl, or lower aliphatic C₁₋₅acyl,
R₅ⱼ is H, monohalomethyl, C₁₋₄alkyl, C₁₋₄alkoxymethyl, C₁₋₄alkyl-thiomethyl, hydroxyethyl, hydroxypropyl, phenyl, aralkyl, C₂₋₄alkenyl or -alkynyl,
each of R₆ⱼ and R₇ⱼ, independently, is H or C₁₋₄alkyl, Xⱼ is O, S, SO or SO₂ and
nⱼ is an integer of 1 to 4,
or a pharmacologically acceptable salt, solvate or hydrate thereof.
- or a compound of formula XIa 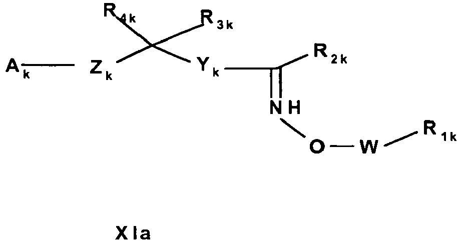 wherein
Aₖ is COOR₅ₖ, OPO(OR₅ₖ)₂, PO(OR₅ₖ)₂, SO₂OR₅ₖ, POR₅ₖOR₅ₖ or 1*H*-tetrazol-5-yl, R₅ₖ being H or C₁₋₆alkyl;
Wₖ is a bond, C₁₋₃alkylene or C₂₋₃alkenylene;
Yₖ is C₆₋₁₀aryl or C₃₋₉heteroaryl, optionally substituted by 1 to 3 radicals selected from halogen, OH, NO₂, C₁₋₆alkyl, C₁₋₆alkoxy; halo-substituted C₁₋₆alkyl and halo-substituted C₁₋₆alkoxy;
Zₖ is azetidine;
R₁ₖ is C₆₋₁₀aryl or C₃₋₉heteroaryl, unsubstituted or substituted by by C₁₋₆alkyl, C₆₋₁₀aryl, C₆₋₁₀arylC₁₋₄alkyl, C₃₋₉heteroaryl, C₃₋₉heteroarylC₁₋₄alkyl, C₃₋₈cycloalkyl, C₃₋₈cycloalkylC₁₋₄alkyl, C₃₋₈heterocycloalkyl or C₃₋₈heterocycloalkylC₁₋₄alkyl;
wherein any aryl, heteroaryl, cycloalkyl or heterocycloalkyl of R₁ₖ is unsubstituted or substituted by 1 to 5 groups selected from halogen, C₁₋₆alkyl, C₁₋₆alkoxy and halo substituted-C₁₋₆alkyl or -C₁₋₆alkoxy;
R₂ₖ is H, C₁₋₆alkyl, halo substituted C₁₋₆alkyl, C₂₋₆alkenyl or C₂₋₆alkynyl: and
each of R₃ₖ or R₄ₖ, independently, is H, halogen, OH, C₁₋₆alkyl, C₁₋₆alkoxy or halo substituted C₁₋₆alkyl or C₁₋₆alkoxy;
and the N-oxide derivatives thereof or prodrugs thereof,
or a pharmacologically acceptable salt, solvate or hydrate thereof.

3. A concentrate according to claim 1 or 2 wherein the S1P receptor modulator or agonist is selected from:
2-amino-2-[2-(4-octylphenylethyl)]propane-1,3-diol;
2-amino-2-{2-[4-(1-oxo-5-phenylpentyl)phenyl]ethyl}propane-1,3-diol;
2-amino-4-(4-heptyloxyphenyl)-2-methyl-butanol;
(2R)-2-amino-4-[3-(4-cyclohexyloxybutyl)-benzo[b]thien-6-yl]-2-methylbutan-1-ol;
2-amino-2-{2-[4-(3-benzyloxyphenoxy)-2-chlorophenyl]ethyl}-1,3-propane-diol;
2-amino-2-{2-[4-(3-benzyloxyphenylthio)-2-chlorophenyl]ethyl}-1,3-propane-diol;
2-amino-{2-[4-(3-benzyloxyphenoxy)-2-chlorophenyl]propyl}-1,3-propane-diol;
2-amino-{2-[4-(3-benzyloxyphenylthio)-2-chlorophenyl]propyl}-1,3-propane-diol;
2-amino-4-[4-(3-benzyloxyphenylthio)-2-chlorophenyl]-2-methylbutane-1-ol;
2-amino-4-[4-(3-benzyloxyphenylthio)-2-chlorophenyl]-2-ethylbutane-1-ol;
1-{4-[1-(4-cyclohexyl-3-trifluoromethyl-benzyloxyimino)-ethyl]-2-ethyl-benzyl}-azetidine-3-carboxylic acid;
or a pharmaceutically acceptable salt or phosphate derivatives thereof.

4. A concentrate according to claim 1 comprising a S1P receptor modulator or agonist selected from 2-amino-2-[2-(4-octylphenylethyl)]propane-1,3-diol, 2-amino-2-[4-(benzyloxyphenylthio)-2-chlorophenyl]ethyl-1,3-propane-diol, or a corresponding phosphate thereof, and 1-{4-[1-(4-cyclohexyl-3-trifluoromethyl-benzyloxyimino)-ethyl]-2-ethyl-benzyl}-azetidine-3-carboxylic acid, or a pharmaceutically acceptable salt thereof.

5. A concentrate according to claim 1 comprising 2-amino-2-[2-(4-octylphenyl-ethyl)]propane-1,3-diol, in free form or in pharmaceutically acceptable salt form or compound of formula IVa 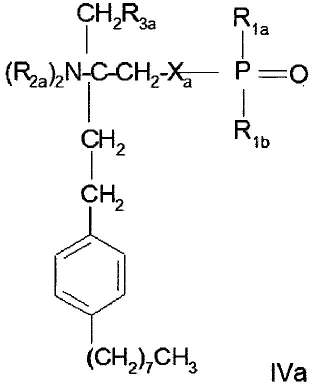 wherein R₂ₐ is H, R₃ₐ is OH, Xₐ is O, R₁ₐ and R_{1b} are OH.

6. A concentrate according to claim 1 comprising 2-amino-2-[2-(4-octylphenyl-ethyl)]propane-1,3-diol in hydrochloride form or compound of formula IVa 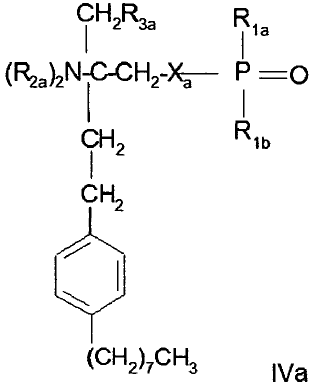 wherein R₂ₐ is H, R₃ₐ is OH, Xₐ is O, R₁ₐ and R_{1b} are OH.

7. A concentrate according to any preceding claim wherein said composition further comprises glycerin.

8. A concentrate according to claim 7 wherein the glycerin and propylene glycol are present in a ratio of 5:95 to 25:75.

9. A concentrate according to any one of claims 1 to 8 which is diluted with a vehicle in a ratio of from 1:1 to more than 1:10.

10. A pharmaceutical solution comprising a concentrate according to any one of claims 1 to 8 diluted with a vehicle in a ratio of from 1:1 to more than 1:10.

11. A pharmaceutical solution according to claim 10 for oral administration.

12. Use of a concentrate according to any one of claims 1 to 8 for the preparation of a medicament for the treatment of a subject in need of immunosuppression.

13. Use of a concentrate according to any one of claims 1 to 8, for the preparation of a medicament for the treatment or prevention of organ or tissue transplant rejection, autoimmune disease or inflammatory conditions.

14. Use according to claim 13 for the preparation of a medicament for treatment and prevention of multiple sclerosis, arthritis, inflammatory bowel diseases, hepatitis, viral myocarditis or viral diseases caused by viral myocarditis.

## Patentansprüche

1. Konzentrat zur Verdünnung, umfassend einen S1P-Rezeptor-Modulator oder -Agonisten oder ein pharmazeutisch unbedenkliches Salz davon und 65 bis 99 Gew.-% Propylenglykol, bezogen auf das Gesamtgewicht der Zusammensetzung.

2. Konzentrat nach Anspruch 1, wobei der S1P-Rezeptor-Modulator oder - Agonist ausgewählt ist aus:
- einer Verbindung der Formel I 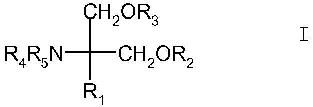 wobei R₁ für
- ein Phenylalkyl, wobei Alkyl eine gerade oder verzweigte (C₆₋₂₀)-Kohlenstoffkette ist; oder
- ein Phenylalkyl, wobei Alkyl eine gerade oder verzweigte (C₁₋₃₀)-Kohlenstoffkette ist, wobei das Phenylalkyl durch
- eine gerade oder verzweigte (C₆₋₂₀)-Kohlenstoffkette, die gegebenenfalls durch Halogen substituiert ist,
- eine gerade oder verzweigte (C₆₋₂₀)-Alkoxykette, die gegebenenfalls durch Halogen substituiert ist,
- ein gerades oder verzweigtes (C₆₋₂₀)-Alkenyloxy,
- Phenyl-C₁₋₁₄-alkoxy, Halogenphenyl-C₁₋₄-alkoxy, Phenyl-C₁₋₁₄-alkoxy-C₁₋₁₄-alkyl, Phenoxy-C₁₋₄-alkoxy oder Phenoxy-C₁₋₄-alkyl,
- Cycloalkylalkyl, das durch C₆₋₂₀-Alkyl substituiert ist,
- Heteroarylalkyl, das durch C₆₋₂₀-Alkyl substituiert ist,
- heterocyclisches C₆₋₂₀-Alkyl oder
- heterocyclisches Alkyl, das durch C₂₋₂₀-Alkyl substituiert ist, substituiert ist,
steht und wobei
die Alkylgruppierung
- in der Kohlenstoffkette eine Bindung oder ein Heteroatom, ausgewählt aus einer Doppelbindung, einer Dreifachbindung, O, S, Sulfinyl, Sulfonyl oder NR₆, wobei R₆ wie oben definiert ist, und
- als Substituent C₁₋₄-Alkoxy, C₂₋₄-Alkenyloxy, C₂₋₄-Alkinyloxy, Aryl-C₁₋₄-alkyloxy, Acyl, C₁₋₄-Alkylamino, C₁₋₄-Alkylthio, Acylamino, (C₁₋₄-Alkoxy)carbonyl, (C₁₋₄-Alkoxy)carbonylamino, Acyloxy, (C₁₋₄-Alkyl)-carbamoyl, Nitro, Halogen, Amino, Hydroxy oder Carboxy aufweisen kann und
R₂, R₃, R₄ und R₅ jeweils unabhängig für H, C₁₋₄-Alkyl oder Acyl stehen,
oder einem pharmazeutisch unbedenklichen Salz oder Hydrat davon;
- einer Verbindung der Formel II 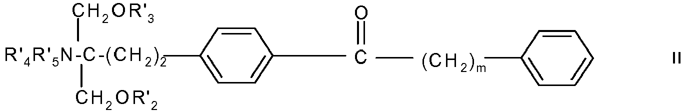
wobei m für 1 bis 9 steht und R'₂, R'₃, R'₄ und R'₅ jeweils unabhängig für H, C₁₋₆-Alkyl oder Acyl stehen,
oder einem pharmazeutisch unbedenklichen Salz oder Hydrat davon;
- einer Verbindung der Formel III 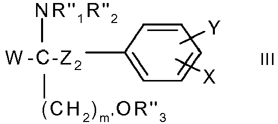
wobei W für H; C₁₋₆-Alkyl, C₂₋₆-Alkenyl oder C₂₋₆-Alkinyl; unsubstituiertes oder durch OH substituiertes Phenyl; R"₄O(CH₂)ₙ oder C₁₋₆-Alkyl, das durch 1 bis 3 Substituenten aus der Gruppe bestehend aus Halogen, C₃₋₈-Cycloalkyl, Phenyl und durch OH substituiertem Phenyl substituiert ist; steht;
X für H oder unsubstituiertes oder substituiertes geradkettiges Alkyl mit einer Zahl p von Kohlenstoffatomen oder unsubstituiert oder substituiert durch 1 bis 3 Substituenten aus der Gruppe bestehend aus C₁₋₆-Alkyl, OH, C₁₋₆-Alkoxy, Acyloxy, Amino, C₁₋₆-Alkylamino, Acylamino, Oxo, Halogen-C₁₋₆-alkyl, Halogen, unsubstituiertem Phenyl und Phenyl, das durch 1 bis 3 Substituenten aus der Gruppe bestehend aus C₁₋₆-Alkyl, OH, C₁₋₆-Alkoxy, Acyl, Acyloxy, Amino, C₁₋₆-Alkylamino, Acylamino, Halogen-C₁₋₆-alkyl und Halogen substituiert ist, steht;
Y für H, C₁₋₆-Alkyl, OH, C₁₋₆-Alkoxy, Acyl, Acyloxy, Amino, C₁₋₆-Alkylamino, Acylamino, Halogen-C₁₋₆-alkyl oder Halogen steht,
Z₂ für eine Einfachbindung oder ein geradkettiges Alkylen mit einer Zahl q von Kohlenstoffatomen steht,
p und q unabhängig für eine ganze Zahl von 1 bis 20 stehen, mit der Maßgabe 6≤p+q≤23, m' für 1, 2 oder 3 steht, n für 2 oder 3 steht, R"₁, R"₂, R"₃ und R"₄ jeweils unabhängig für H, C₁₋₄-Alkyl oder Acyl stehen,
oder einem pharmazeutisch unbedenklichen Salz oder Hydrat davon;
- einer Verbindung der Formel IVa oder IVb 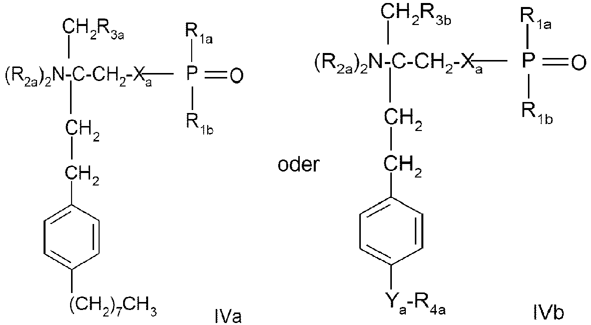
wobei Xₐ für O, S, NR₁ₛ oder eine Gruppe -(CH₂)ₙₐ-, wobei die Gruppe unsubstituiert oder durch 1 bis 4 Halogen substituiert ist, steht;
nₐ für 1 oder 2 steht, R₁ₛ für H oder (C₁₋₄)-Alkyl, wobei das Alkyl unsubstituiert oder durch Halogen substituiert ist, steht;
R₁ₐ für H, OH, (C₁₋₄)-Alkyl oder O-(C₁₋ₐ)-Alkyl, wobei Alkyl unsubstituiert oder durch 1 bis 3 Halogen substituiert ist, steht;
R_{1b} für H, OH oder (C₁₋₄)-Alkyl, wobei Alkyl unsubstituiert oder durch Halogen substituiert ist, steht;
R₂ₐ jeweils unabhängig aus H oder (C₁₋₄)-Alkyl, wobei Alkyl unsubstituiert oder durch Halogen substituiert ist, ausgewählt ist;
R₃ₐ für H, OH, Halogen oder O-(C₁₋₄)-Alkyl, wobei Alkyl unsubstituiert oder durch Halogen substituiert ist, steht; und R_{3b} für H, OH, Halogen, (C₁₋₄)-Alkyl, wobei Alkyl unsubstituiert oder durch Hydroxyl substituiert ist, oder O-(C₁₋₄)-Alkyl, wobei Alkyl unsubstituiert oder durch Halogen substituiert ist, steht;
Yₐ für -CH₂-, -C(O)-, -CH(OH)-, -C(=NOH)-, O oder S steht und R₄ₐ für (C₄₋₁₄)-Alkyl oder (C₄₋₁₄)-Alkenyl steht;
oder einem pharmazeutisch unbedenklichen Salz oder Hydrat davon;
- einer Verbindung der Formel VII 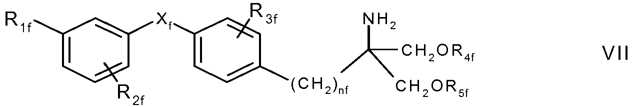
wobei X_{f} für O, S, SO oder SO₂ steht,
R_{1f} für Halogen, Trihalogenmethyl, OH, C₁₋₇-Alkyl, C₁₋₄-Alkoxy, Trifluormethoxy, Phenoxy, Cyclohexylmethyloxy, Pyridylmethoxy, Cinnamyloxy, Naphthylmethoxy, Phenoxymethyl, CH₂-OH, CH₂-CH₂-OH, C₁₋₄-Alkylthio, C₁₋₄-Alkylsulfinyl, C₁₋₄-Alkylsulfonyl, Benzylthio, Acetyl, Nitro oder Cyano oder Phenyl, Phenyl-C₁₋₄-alkyl oder Phenyl-C₁₋₄-alkoxy, wobei jede Phenylgruppe davon unsubstituiert oder durch Halogen, CF₃, C₁₋₄-Alkyl oder C₁₋₄-Alkoxy substituiert ist, steht;
R_{2f} für H, Halogen, Trihalogenmethyl, C₁₋₄-Alkoxy, C₁₋₇-Alkyl, Phenethyl oder Benzyloxy steht;
R_{3f} für H, Halogen, CF₃, OH, C₁₋₇-Alkyl, C₁₋₄-Alkoxy, Benzyloxy oder C₁₋₄-Alkoxymethyl steht;
R_{4f} und R_{5f} jeweils unabhängig für H oder einen Rest der Formel 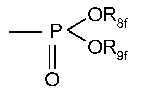
stehen, wobei R_{8f} und R_{9f} jeweils unabhängig für H oder C₁₋₄-Alkyl, das unsubstituiert oder durch Halogen substituiert ist, stehen;
und n_{f} für eine ganze Zahl von 1 bis 4 steht;
oder einem pharmakologischen Salz, Solvat oder Hydrat davon;
- einer Verbindung der Formel X 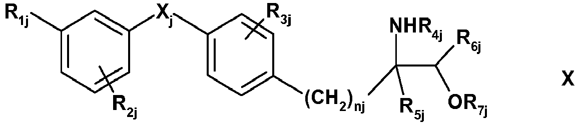 wobei
R₁ⱼ für Halogen, Trihalogenmethyl, C₁₋₄-Alkyl, C₁₋₄-Alkoxy, C₁₋₄-Alkylthio, C₁₋₄-Alkylsulfinyl, C₁₋₄-Alkylsulfonyl, Aralkyl, unsubstituiert oder substituiert durch Phenoxy oder Aralkyloxy, steht,
R₂ⱼ für H, Halogen, Trihalogenmethyl, C₁₋₄-Alkyl, C₁₋₄-Alkoxy, Aralkyl oder Aralkyloxy steht,
R₃ⱼ für H, Halogen, CF₃, C₁₋₄-Alkyl, C₁₋₄-Alkoxy, C₁₋₄-Alkylthio oder Benzyloxy steht,
R₄ⱼ für H, C₁₋₄-Alkyl, Phenyl, unsubstituiert oder durch Benzyl oder Benzoyl substituiert, oder niederaliphatisches C₁₋₅-Acyl steht,
R₅ⱼ für H, Monohalogenmethyl, C₁₋₄-Alkyl, C₁₋₄-Alkoxymethyl, C₁₋₄-Alkylthiomethyl, Hydroxyethyl, Hydroxypropyl, Phenyl, Aralkyl, C₂₋₄-Alkenyl oder -Alkinyl steht,
R₆ⱼ und R₇ⱼ jeweils unabhängig für H oder C₁₋₄-Alkyl stehen, Xⱼ für O, S, SO oder SO₂ steht und
nⱼ für eine ganze Zahl von 1 bis 4 steht,
oder einem pharmakologisch unbedenklichen Salz, Solvat oder Hydrat davon;
- oder einer Verbindung der Formel XIa 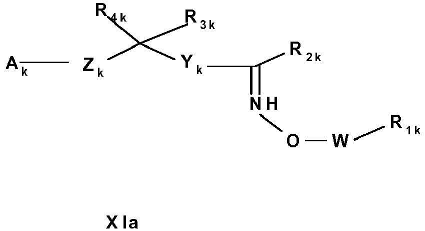 wobei
Aₖ für COOR₅ₖ, OPO(OR₅ₖ)₂, PO(OR₅ₖ)₂, SO₂OR₅ₖ, POR₅ₖOR₅ₖ oder 1*H-*Tetrazol-5-yl steht, wobei R₅ₖ für H oder C₁₋₆-Alkyl steht;
Wₖ für eine Bindung, C₁₋₃-Alkylen oder C₂₋₃-Alkenylen steht;
Yₖ für C₆₋₁₀-Aryl oder C₃₋₉-Heteroaryl, gegebenenfalls substituiert durch 1 bis 3 Reste, die aus Halogen, OH, NO₂, C₁₋₆-Alkyl, C₁₋₆-Alkoxy, halogensubstituiertem C₁₋₆-Alkyl und halogensubstituiertem C₁₋₆-Alkoxy ausgewählt sind, steht;
Zₖ für Azetidin steht;
R₁ₖ für C₆₋₁₀-Aryl oder C₃₋₉-Heteroaryl, unsubstituiert oder durch C₁₋₆-Alkyl, C₆₋₁₀-Aryl, C₆₋₁₀-Aryl-C₁₋₄-alkyl, C₃₋₉-Heteroaryl, C₃₋₉-Heteroaryl-C₁₋₄-alkyl, C₃₋₈-Cycloalkyl, C₃₋₈-Cycloalkyl-C₁₋₄alkyl, C₃₋₈-Heterocycloalkyl oder C₃₋₈-Heterocycloalkyl-C₁₋₄-alkyl substituiert, steht; wobei jedes Aryl, Heteroaryl, Cycloalkyl oder Heterocycloalkyl von R₁ₖ unsubstituiert oder durch 1 bis 5 Gruppen, die aus Halogen, C₁₋₆-Alkyl, C₁₋₆-Alkoxy und halogensubstituiertem C₁₋₆-Alkyl oder C₁₋₆-Alkoxy ausgewählt sind, substituiert ist;
R₂ₖ für H, C₁₋₆-Alkyl, halogensubstituiertes C₁₋₆-Alkyl, C₂₋₆-Alkenyl oder C₂₋₆-Alkinyl steht; und
R₃ₖ oder R₄ₖ jeweils unabhängig für H, Halogen, OH, C₁₋₆-Alkyl, C₁₋₆-Alkoxy oder halogensubstituiertes C₁₋₆-Alkyl oder C₁₋₆-Alkoxy steht; und den N-Oxid-Derivaten davon oder Prodrugs davon,
oder einem pharmakologisch unbedenklichen Salz, Solvat oder Hydrat davon.

3. Konzentrat nach Anspruch 1 oder 2, wobei der S1P-Rezeptor-Modulator oder -Agonist ausgewählt ist aus:
2-Amino-2-[2-(4-octylphenylethyl)]propan-1,3-diol;
2-Amino-2-{2-[4-(1-oxo-5-phenylpentyl)phenyl]ethyl}propan-1,3-diol;
2-Amino-4-(4-heptyloxyphenyl)-2-methylbutanol;
(2R)-2-Amino-4-[3-(4-cyclohexyloxybutyl)benzo[b]thien-6-yl]-2-methylbutan-1-ol;
2-Amino-2-{2-[4-(3-benzyloxyphenoxy)-2-chlorphenyl]ethyl}-1,3-propandiol;
2-Amino-2-{2-[4-(3-benzyloxyphenylthio)-2-chlorphenyl]ethyl}-1,3-propandiol;
2-Amino-{2-[4-(3-benzyloxyphenoxy)-2-chlorphenyl]propyl}-1,3-propandiol;
2-Amino-{2-[4-(3-benzyloxyphenylthio)-2-chlorphenyl]propyl}-1,3-propandiol;
2-Amino-4-[4-(3-benzyloxyphenylthio)-2-chlorphenyl]-2-methylbutan-1-ol;
2-Amino-4-[4-(3-benzyloxyphenylthio)-2-chlorphenyl]-2-ethylbutan-1-ol;
1-{4-[1-(4-Cyclohexyl-3-trifluormethylbenzyloxyimino)ethyl]-2-ethylbenzyl}azetidin-3 -carbonsäure;
oder einem pharmazeutisch unbedenklichen Salz oder Phosphatderivaten davon.

4. Konzentrat nach Anspruch 1, umfassend einen S1P-Rezeptor-Modulator oder -Agonisten, der ausgewählt ist aus 2-Amino-2-[2-(4-octylphenylethyl)]propan-1,3-diol, 2-Amino-2-[4-(benzyloxyphenylthio)-2-chlorphenyl]ethyl-1,3-propandiol oder einem entsprechenden Phosphat davon und 1-{4-[1-(4-Cyclohexyl-3-trifluormethylbenzyloxyimino)ethyl]-2-ethylbenzyl}azetidin-3-carbonsäure oder einem pharmazeutisch unbedenklichen Salz davon.

5. Konzentrat nach Anspruch 1, umfassend 2-Amino-2-[2-(4-octylphenylethyl)]propan-1,3-diol in freier Form oder in pharmazeutisch unbedenklicher Salzform oder Verbindung der Formel IVa 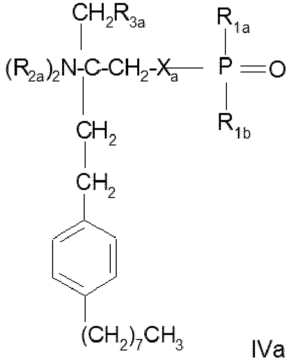 wobei R₂ₐ für H steht, R₃ₐ für OH steht, Xₐ für O steht, R₁ₐ und R_{1b} für OH stehen.

6. Konzentrat nach Anspruch 1, umfassend 2-Amino-2-[2-(4-octylphenylethyl)]propan-1,3-diol in Hydrochloridform oder Verbindung der Formel IVa 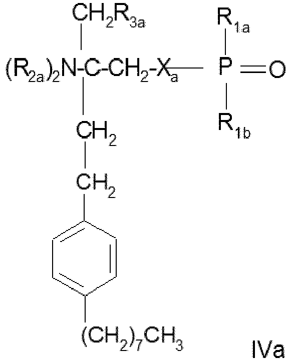 wobei R₂ₐ für H steht, R₃ₐ für OH steht, Xₐ für O steht, R₁ₐ und R_{1b} für OH stehen.

7. Konzentrat nach einem der vorhergehenden Ansprüche, wobei die Zusammensetzung ferner Glycerin umfasst.

8. Konzentrat nach Anspruch 7, wobei das Glycerin und das Propylenglykol in einem Verhältnis von 5:95 bis 25:75 vorliegen.

9. Konzentrat nach einem der Ansprüche 1 bis 8, das in einem Verhältnis von 1:1 bis mehr als 1:10 mit einem Vehikel verdünnt ist.

10. Pharmazeutische Lösung, umfassend ein Konzentrat nach einem der Ansprüche 1 bis 8, das in einem Verhältnis von 1:1 bis mehr als 1:10 mit einem Vehikel verdünnt ist.

11. Pharmazeutische Lösung nach Anspruch 10 zur oralen Verabreichung.

12. Verwendung eines Konzentrats nach einem der Ansprüche 1 bis 8 zur Herstellung eines Medikaments zur Behandlung eines Empfängers, der Immunsuppression bedarf.

13. Verwendung eines Konzentrats nach einem der Ansprüche 1 bis 8 zur Herstellung eines Medikaments zur Behandlung oder Prävention von Organ- oder Gewebetransplantatabstoßung, Autoimmunerkrankung oder entzündlichen Leiden.

14. Verwendung nach Anspruch 13 zur Herstellung eines Medikaments zur Behandlung oder Prävention von multipler Sklerose, Arthritis, entzündlichen Darmerkrankungen, Hepatitis, Virusmyokarditis oder durch Virusmyokarditis verursachten Viruserkrankungen.

## Revendications

1. Concentré pour dilution comprenant un agoniste ou modulateur de récepteur S1P, ou sel pharmaceutiquement acceptable de celui-ci, et 65 à 99 % en masse de propylène glycol, par rapport à la masse totale de la composition.

2. Concentré selon la revendication 1, où l'agoniste ou modulateur de récepteur S1P est choisi parmi :
- un composé de formule I 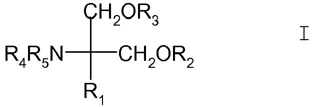 où R₁ représente
- un groupement phénylalkyle où alkyle désigne une chaîne carbonée en C₆₋₂₀ linéaire ou ramifiée ; ou
- un groupement phénylalkyle où alkyle désigne une chaîne carbonée en C₁₋₃₀ linéaire ou ramifiée, où ledit groupement phénylalkyle est substitué par
- une chaîne carbonée en C₆₋₂₀ linéaire ou ramifiée éventuellement substituée par halogène,
- une chaîne alkoxy en C₆₋₂₀ linéaire ou ramifiée éventuellement substituée par halogène,
- une chaîne alcényloxy en C₆₋₂₀ linéaire ou ramifiée,
- un groupement phényl-(alkoxy en C₁₋₁₄), halogénophényl-(alkoxy en C₁₋₄), phényl-(alkoxy en C₁₋₁₄)-(alkyle en C₁₋₁₄), phénoxy-(alkoxy en C₁₋₄) ou phénoxy-(alkyle en C₁₋₄),
- cycloalkylalkyle substitué par alkyle en C₆₋₂₀,
- hétéroarylalkyle substitué par alkyle en C₆₋₂₀,
- alkyle hétérocyclique en C₆₋₂₀ ou
- alkyle hétérocyclique substitué par alkyle en C₂₋₂₀,
et où
l'entité alkyle peut comporter
- dans la chaîne carbonée, une liaison ou un hétéroatome choisis parmi une double liaison, une triple liaison, O, S, sulfinyle, sulfonyle ou NR₆, où R₆ est tel que défini ci-avant, et
- en tant que substituant, alkoxy en C₁₋₄, alcényloxy en C₂₋₄, alcynyloxy en C₂₋₄, aryl-(alkoxy en C₁₋₄), acyle, (alkyle en C₁₋₄)-amino, (alkyle en C₁₋₄)-thio, acylamino, (alkoxy en C₁₋₄)-carbonyle, (alkoxy en C₁₋₄)-carbonylamino, acyloxy, (alkyle en C₁₋₄)-carbamoyle, nitro, halogène, amino, hydroxy ou carboxy, et chacun des radicaux R₂, R₃, R₄ et R₅ représente indépendamment H ou un groupement alkyle en C₁₋₄ ou acyle
ou l'un des sels ou hydrates pharmaceutiquement acceptables de celui-ci ;
- un composé de formule II 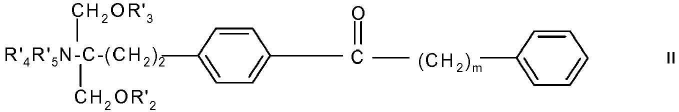
où m est compris entre 1 et 9 et chacun des radicaux R'₂, R'₃, R'₄ et R'₅ représente indépendamment H ou un groupement alkyle en C₁₋₆ ou acyle,
ou l'un des sels ou hydrates pharmaceutiquement acceptables de celui-ci ;
- un composé de formule III 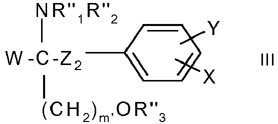
où W représente H ; un groupement alkyle en C₁₋₆, alcényle en C₂₋₆ ou alcynyle en C₂₋₆, phényle non substitué ou substitué par OH ; R"₄O(CH₂)ₙ; ou alkyle en C₁₋₆ substitué par 1 à 3 substituants choisis dans le groupe constitué par les atomes d'halogène et les groupements cycloalkyle en C₃₋₈, phényle et phényle substitué par OH;
X représente H ou un groupement alkyle à chaîne linéaire non substituée ou substituée comportant un nombre p d'atomes de carbone ou non substituée ou substituée par 1 à 3 substituants choisis dans le groupe constitué par les groupements alkyle en C₁₋₆, OH, alkoxy en C₁₋₆, acyloxy, amino, (alkyle en C₁₋₆)-amino, acylamino, oxo, halogéno-(alkyle en C₁₋₆), les atomes d'halogène, les groupements phényle non substitué et phényle substitué par 1 à 3 substituants choisis dans le groupe constitué par les groupements alkyle en C₁₋₆, OH, alkoxy en C₁₋₆, acyle, acyloxy, amino, (alkyle en C₁₋₆)-amino, acylamino, halogéno-(alkyle en C₁₋₆) et les atomes d'halogène ;
Y représente H ou un groupement alkyle en C₁₋₆, OH, alkoxy en C₁₋₆, acyle, acyloxy, amino, (alkyle en C₁₋₆)-amino, acylamino, halogéno-(alkyle en C₁₋₆) ou un atome d'halogène,
Z₂ représente une liaison simple ou un groupement alkylène à chaîne linéaire comportant q atomes de carbone,
chacun des nombres p et q est indépendamment égal à un entier compris entre 1 et 20, à la condition que 6 ≤ p + q ≤ 23, m' est égal à 1, 2 ou 3, n est égal à 2 ou 3, chacun des radicaux R"₁, R"₂, R"₃ et R"₄ représente indépendamment H ou un groupement alkyle en C₁₋₄ ou acyle,
ou l'un des sels ou hydrates pharmaceutiquement acceptables de celui-ci,
- un composé de formule IVa ou IVb 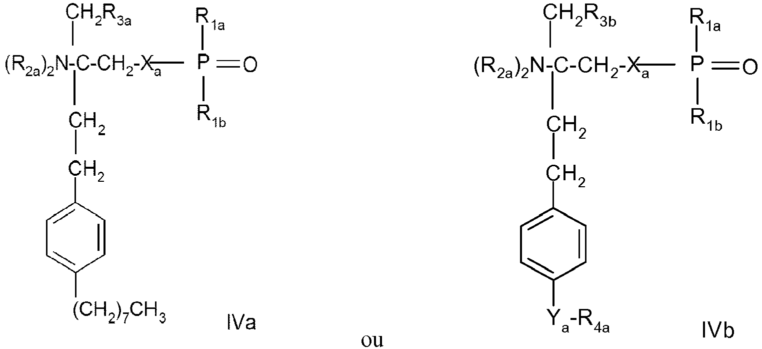
où Xₐ représente O, S, NR₁ₛ ou un groupement -(CH₂)ₙₐ-, ledit groupement étant non substitué ou substitué par 1 à 4 atomes d'halogène ;
nₐ est égal à 1 ou à 2, R₁ₛ représente H ou un groupement alkyle en C₁₋₄, ledit groupement alkyle étant non substitué ou substitué par halogène ;
R₁ₐ représente H ou un groupement OH, alkyle en C₁₋₄ ou O-(alkyle en C₁₋₄) où le groupement alkyle est non substitué ou substitué par 1 à 3 atomes d'halogène ;
R_{1b} représente H ou un groupement OH ou alkyle en C₁₋₄, où le groupement alkyle est non substitué ou substitué par halogène ;
chacun des radicaux R₂ₐ est indépendamment choisi parmi H ou les groupements alkyle en C₁₋₄, ledit groupement alkyle étant non substitué ou substitué par halogène ;
R₃ₐ représente H ou un groupement OH, un atome d'halogène ou un groupement O-(alkyle en C₁₋₄), où le groupement alkyle est non substitué ou substitué par halogène ; et R_{3b} représente H ou un groupement OH, un atome d'halogène ou un groupement alkyle en C₁₋₄, où le groupement alkyle est non substitué ou substitué par hydroxy, ou O-(alkyle en C₁₋₄), où le groupement alkyle est non substitué ou substitué par halogène ;
Yₐ représente -CH₂-, -C(O)-, -CH(OH)-, -C(=NOH)-, O ou S,
et R₄ₐ représente un groupement alkyle en C₄₋₁₄ ou alcényle en C₄₋₁₄;
ou l'un des sels ou hydrates pharmaceutiquement acceptables de celui-ci ;
- un composé de formule VII 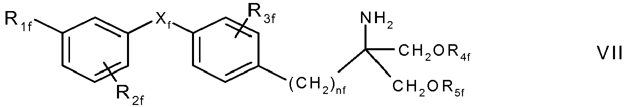
où X_{f} représente O, S, SO ou SO₂
R_{1f} représente un atome d'halogène ou un groupement trihalogénométhyle, OH, alkyle en C₁₋₇, alkoxy en C₁₋₄, trifluorométhoxy, phénoxy, cyclohexylméthyloxy, pyridylméthoxy, cinnamyloxy, naphtylméthoxy, phénoxyméthyle, CH₂-OH, CH₂-CH₂-OH, (alkyle en C₁₋₄)-thio, (alkyle en C₁₋₄)-sulfinyle, (alkyle en C₁₋₄)-sulfonyle, benzylthio, acétyle, nitro ou cyano, ou phényle, phényl-(alkyle en C₁₋₄) ou phényl-(alkoxy en C₁₋₄), chacun des groupements phényle de celui-ci étant non substitué ou substitué par halogène, CF₃, alkyle en C₁₋₄ ou alkoxy en C₁₋₄ ;
R_{2f} représente H, un atome d'halogène ou un groupement trihalogénométhyle, alkoxy en C₁₋₄, alkyle en C₁₋₇, phénéthyle ou benzyloxy ;
R_{3f} représente H, un atome d'halogène ou un groupement CF₃, OH, alkyle en C₁₋₇, alkoxy en C₁₋₄, benzyloxy ou (alkoxy en C₁₋₄)-méthyle ;
chacun des radicaux R_{4f} et R_{5f} représente indépendamment H ou un résidu de formule 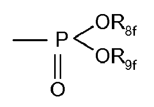
où chacun des radicaux R_{8f} et R_{9f} représente indépendamment H ou un groupement alkyle en C₁-₄ non substitué ou substitué par halogène ;
et n_{f} représente un entier compris entre 1 et 4 ;
ou un sel, solvate ou hydrate pharmacologique de celui-ci ;
- un composé de formule X 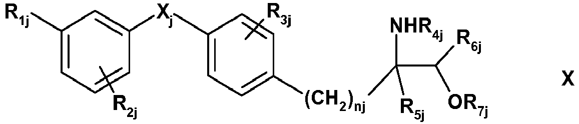 où
R₁ⱼ représente un atome d'halogène ou un groupement trihalogénométhyle, alkyle en C₁₋₄, alkoxy en C₁₋₄, (alkyle en C₁₋₄)-thio, (alkyle en C₁₋₄)-sulfinyle, (alkyle en C₁₋₄)-sulfonyle, arylalkyle, non substitué ou substitué par phénoxy ou arylalkyloxy, R₂ⱼ représente H, un atome d'halogène ou un groupement trihalogénométhyle, alkyle en C₁₋₄, alkoxy en C₁₋₄, arylalkyle ou aralkyloxy,
R₃ⱼ représente H, un atome d'halogène ou un groupement CF₃, alkyle en C₁₋₄, alkoxy en C₁₋₄, (alkyle en C₁₋₄)-thio ou benzyloxy,
R₄ⱼ représente H ou un groupement alkyle en C₁₋₄, phényle, non substitué ou substitué par benzyle ou benzoyle, ou acyle aliphatique court en C₁₋₅,
R₅ⱼ représente H ou un groupement monohalogénométhyle, alkyle en C₁₋₄, (alkoxy en C₁₋₄)-méthyle, (alkyle en C₁₋₄)-thiométhyle, hydroxyéthyle, hydroxypropyle, phényle, arylalkyle, alcényle en C₂₋₄ ou alcynyle en C₂₋₄,
chacun des radicaux R₆ⱼ et R₇ⱼ représente indépendamment H ou un groupement alkyle en C₁₋₄, Xⱼ représente O, S, SO ou SO₂ et
nⱼ représente un entier compris entre 1 et 4,
ou l'un des sels, solvates ou hydrates pharmacologiquement acceptables de celui-ci.
- ou un composé de formule XIa 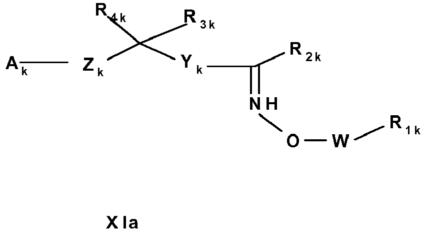 où
Aₖ représente COOR₅ₖ, OPO(OR₅ₖ)₂, PO(OR₅ₖ)₂, SO₂OR₅ₖ, POR₅ₖOR₅ₖ ou un groupement 1*H*-tétrazol-5-yle, R₅ₖ représentant H ou un groupement alkyle en C₁₋₆;
Wₖ représente une liaison ou un groupement alkylène en C₁₋₃ ou alcénylène en C₂₋₃ ;
Yₖ représente un groupement aryle en C₆₋₁₀ ou hétéroaryle en C₃₋₉, éventuellement substitué par 1 à 3 radicaux choisis parmi les atomes d'halogène et les groupements OH, NO₂, alkyle en C₁₋₆, alkoxy en C₁₋₆ ; halogénoalkyle en C₁₋₆alkyl et halogénoalkoxy en C₁₋₆ ;
Zₖ représente un groupement azétidine ;
R₁ₖ représente un groupement aryle en C₆₋₁₀ ou hétéroaryle en C₃₋₉, non substitué ou substitué par alkyle en C₁₋₆, aryle en C₆₋₁₀, (aryle en C₆₋₁₀)-(alkyle en C₁₋₄), hétéroaryle en C₃₋₉, (hétéroaryle en C₃₋₉)-(alkyle en C₁₋₄), cycloalkyle en C₃₋₈, (cycloalkyle en C₃₋₈)-(alkyle en C₁₋₄),
hétérocycloalkyle en C₃₋₈ ou (hétérocycloalkyle en C₃₋₈)-(alkyle en C₁₋₄) ;
où chacun des groupements aryle, hétéroaryle, cycloalkyle ou hétérocycloalkyle de R₁ₖ est non substitué ou substitué par 1 à 5 groupements choisis parmi les atomes d'halogène et les groupements alkyle en C₁₋₆, alkoxy en C₁₋₆ et halogénoalkyle en C₁₋₆ ou halogénoalkoxy en C₁₋₆ ;
R₂ₖ représente H ou un groupement alkyle en C₁₋₆, halogénoalkyle en C₁₋₆, alcényle en C₂₋₆ ou alcynyle en C₁₋₆ ; et
chacun des radicaux R₃ₖ ou R₄ₖ représente indépendamment H, un atome d'halogène ou un groupement OH, alkyle en C₁₋₆, alkoxy en C₁₋₆ ou halogénoalkyle en C₁₋₆ ou halogénoalkoxy en C₁₋₆ ;
et les dérivés N-oxydes de celui-ci ou les promédicaments de celui-ci,
ou l'un des sels, solvates ou hydrates pharmacologiquement acceptables de celui-ci.

3. Concentré selon la revendication 1 ou 2, où l'agoniste ou modulateur de récepteur S1P est choisi parmi les suivants :
2-amino-2-[2-(4-octylphényléthyl)]propane-1,3-diol ;
2-amino-2-{2-[4-(1-oxo-5-phénylpentyl)phényl]éthyl}propane-1,3-diol ;
2-amino-4-(4-heptyloxyphényl)-2-méthyl-butanol ;
(2R)-2-amino-4-[3-(4-cyclohexyloxybutyl)-benzo[b]thién-6-yl]-2-méthylbutan-1-ol ;
2-amino-2-{2-[4-(3-benzyloxyphénoxy)-2-chlorophényl]éthyl}-1,3-propane-diol ;
2-amino-2-{2-[4-(3-benzyloxyphénylthio)-2-chlorophényl]éthyl}-1,3-propane-diol ;
2-amino-{2-[4-(3-benzyloxyphénoxy)-2-chlorophényl]propyl}-1,3-propane-diol ;
2-amino-{2-[4-(3-benzyloxyphénylthio)-2-chlorophényl]propyl}-1,3-propane-diol ;
2-amino-4-[4-(3-benzyloxyphénylthio)-2-chlorophényl]-2-méthylbutan-1-ol ;
2-amino-4-[4-(3-benzyloxyphénylthio)-2-chlorophényl]-2-éthylbutan-1-ol ;
acide 1-{4-[1-(4-cyclohexyl-3-trifluorométhyl-benzyloxyimino)-éthyl]-2-éthyl-benzyl}-azétidine-3-carboxylique ;
ou l'un des sels ou dérivés phosphates pharmaceutiquement acceptable de celui-ci.

4. Concentré selon la revendication 1 comprenant un agoniste ou modulateur de récepteur S1P choisi parmi les suivants : 2-amino-2-[2-(4-octyl-phényléthyl)]propane-1,3-diol, 2-amino-2-[4-(benzyloxyphénylthio)-2-chlorophényl]éthyl-1,3-propane-diol, ou l'un des phosphates correspondants de ceux-ci, et acide 1-{4-[1-(4-cyclohexyl-3-trifluorométhyl-benzyloxyimino)-éthyl]-2-éthyl-benzyl}-azétidine-3-carboxylique, ou l'un des sels pharmaceutiquement acceptables de celui-ci.

5. Concentré selon la revendication 1, comprenant du 2-amino-2-[2-(4-octylphényléthyl)]propane-1,3-diol, sous forme libre ou sous forme d'un sel pharmaceutiquement acceptable ou un composé de formule IVa 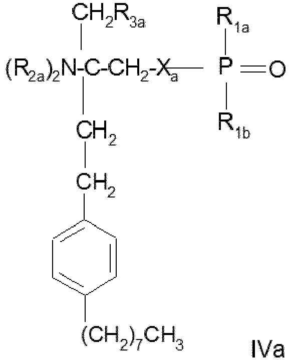 où R₂ₐ représente H, R₃ₐ représente OH, Xₐ représente O, R₁ₐ et R_{1b} représentent OH.

6. Concentré selon la revendication 1, comprenant du 2-amino-2-[2-(4-octylphényléthyl)]propane-1,3-diol sous forme de chlorhydrate ou un composé de formule IVa 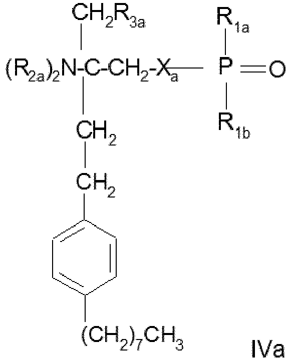 où R₂ₐ représente H, R₃ₐ représente OH, Xₐ représente O, R₁ₐ et R_{1b} représentent OH.

7. Concentré selon l'une quelconque des revendications précédentes, où ladite composition comprend en outre de la glycérine.

8. Concentré selon la revendication 7, où la glycérine et le propylène glycol sont présents dans un rapport de 5:95 à 25:75.

9. Concentré selon l'une quelconque des revendications 1 à 8, qui est dilué par un vecteur dans un rapport compris entre 1:1 et plus de 1:10.

10. Solution pharmaceutique comprenant un concentré selon l'une quelconque des revendications 1 à 8, dilué par un vecteur dans un rapport compris entre 1:1 et plus de 1:10.

11. Solution pharmaceutique selon la revendication 10 pour administration orale.

12. Utilisation d'un concentré selon l'une quelconque des revendications 1 à 8 dans l'élaboration d'un médicament destiné au traitement d'un sujet nécessitant une immunosuppression.

13. Utilisation d'un concentré selon l'une quelconque des revendications 1 à 8 dans l'élaboration d'un médicament destiné au traitement prophylactique ou thérapeutique d'un rejet de greffon d'organe ou de tissu, d'une maladie auto-immune ou d'états inflammatoires.

14. Utilisation selon la revendication 13 dans l'élaboration d'un médicament destiné au traitement prophylactique ou thérapeutique de la sclérose en plaques, de l'arthrite, des maladies inflammatoires chroniques de l'intestin, de l'hépatite, de la myocardite virale ou des maladies virales provoquées par la myocardite virale.
